Europäisches Patentamt

European Patent Office

Office européen des brevets

(11) Publication number: **0 262 971**
**A2**

(12) **EUROPEAN PATENT APPLICATION**

(21) Application number: **87308718.3**

(22) Date of filing: **01.10.87**

(51) Int. Cl.⁴: **A 01 H 1/02**
**C 12 N 15/00**

(30) Priority: **01.10.86 US 913911**

(43) Date of publication of application:
**06.04.88 Bulletin 88/14**

(84) Designated Contracting States:
**AT BE CH DE ES FR GB GR IT LI LU NL SE**

(71) Applicant: **THE PLANT CELL RESEARCH INSTITUTE INC.**
**6560 Trinity Court**
**Dublin California 94568 (US)**

(72) Inventor: **Trulson, Anna J.**
**7000 Tesla Road**
**Livermore California 94550 (US)**

**Shaheen, Elias A.**
**2929 Trotter Way**
**Walnut Creek California 94596 (US)**

(74) Representative: **Goldin, Douglas Michael et al**
**J.A. KEMP & CO. 14, South Square Gray's Inn**
**London WC1R 5EU (GB)**

(54) Methods for controlled regeneration of Cucumis sp. plants in vitro from explant tissue.

(57) Generation of competent embryoids, plantlets and plants (including seeds) from Cucumis sp. explant tissue, e.g., from cotyledonary, root, leaf or stem tissue, by induction followed by promotion of embryoid development in a series of media containing one of more of 2,4-D, BAP and NAA under defined temperature, time and light conditions is disclosed. The explant tissue may be from any plant type: wild or cultivated, hybrid (somatic or sexual), or genetically variant or transformed plants. Competent embryoids may be continuously produced by serial subculture. Serial transfer to novel induction and expression media for defined periods causes sequential development of competent embryoids and plantlets. Transfer to development media permits plantlet development, which in turn progress to mature plants in hardening media and/or soil. The process is applicable to producing encapsulated embryoids from single genotypes or somatic hybrids as "artificial seeds". The process applied to increase of F-1 hybrids permits production of "artificial seeds" (encapsulations) in 1/2 to 1/10 the time it would take to produce natural hybrid seeds. Techniques involving freeze storage and cell suspensions are dislcosed. All representative cultivars were regenerated.

EP 0 262 971 A2

**Description**

METHODS FOR CONTROLLED REGENERATION OF CUCUMIS SP. PLANTS IN VITRO FROM EXPLANT
TISSUE

Cross Reference to Related Applications:

The methods and media techniques of this invention or of our copending protoplast regeneration case (our reference N.43004, hereinafter referred to as 92/13)filed of even date herewith may be preceeded by genetic transformation with Agrobacterium rhizogenes as set forth in our copending case (our reference N.43005 hereinafter referred to as 92/12) filed of even date herewith. Likewise somaclonal variants produced by the techniques of this case, or somatic hybrids produced by protoplast fusion (case 92/13) may be regenerated by the techniques of this case or transformed by the techniques of case 92/12. The disclosures of those cases are hereby incorporated by reference herein to the extent needed.

Field

The present invention relates to controlled regeneration of Cucumis sp. plants in vitro. More specifically, this invention relates to methods of regeneration of plants from embryoids induced from plant explants, which embryoids are fully capable of completing development into mature plants, by use of solid (agar) or liquid cell suspension culture techniques employing a novel series of media for induction and maturation of the embryoids. Serial suculturing permits relatively continuous production of secondary, tertiary, etc. embryoids which can be used for encapsulation as "artificial seeds". This is particularly advantageous in the case of hybridization, where use of hybrid explant tissue from a natural F-1 hybrid can produce such embryoids for encapsulation as artificial seeds in a much shorter time than required for the full cycle of plant maturation and seed development.

Background

Sterility barriers between species are among the most limiting factors in plant breeding. They preclude transfer of many desirable traits such as disease, insect and herbicide resistance between species of cultivated or weed plants because of sexual incompatibility. This problem is especially acute in the family Cucurbitaceae, which includes the genera Cucumis (cucumbers [C. sativus], and melons [C. melo]), Citrollus (watermelons) and Cucurbita (squash and pumpkins). In the genus Cucumis, successful sexual crosses can be made only between C. sativus and the closely related C. harwickii. Attempts to interbreed cucumbers and other species in this genus failed (Deakin et al., 1971). The recent advances in genetic engineering seem to be a promising alternative to sexual propagation techniques for improvement of this economically important crop. However, the lack of a reliable method for regeneration of cucumis plants by tissue culture techniques has prevented significant progress (Lazarte and Sasser, 1982; Malepszy and Nadolska-Orczyk 1983; Novak and Dolezelova 1982; Sekioka and Tanaka, 1981; Kim and Jang, 1984; Wehner and Locy, 1981).

Numerous examples of application of tissue culture technology in plant breeding have been published so far. Novel selection schemes have been devised and effectively used in improvement of important food crops by increasing their resistance to diseases, herbicides and other toxic substances via selection in tissue culture (Carlson, 1973; Chaleff and Parsons, 1978; for application of tissue culture in breeding for disease resistance see review by Wenzel, 1985). The increase in genetic variation that led to this improvement was due to the phenomenon of somaclonal variation that is frequently associated with tissue culture (Larkin and Scowcroft, 1981). The perhaps most important aspect of the tissue culture technology is the application in genetic engineering of plants. Polyethylene oxide solutions have been used to coat asexual embryos of carrot (Daucus carota L.) to form synthetic seed coats (Kitto and Janick, 1985).

So far, the Cucurbitaceae resisted application of tissue culture. Although some success in in vitro induction of embryogenesis in cucumbers (Novak and Dolezalova, 1982; Wehner and Locy, 1981; Malepszy and Nadolska-Orczyk, 1983) and pumpkins (Jelaska, 1972, 1974) and regeneration of muskmelons (Moreno et al., 1985) was reported, the lack of reliable regeneration procedures for majority of the members in this family precludes wide application of novel technologies to their breeding. Application of tissue culture is particularly important in cucumber breeding, since genetic variability of this economically important crop is limited due to sexual incompatibility with other species and genera in the Cucurbitaceae (Deakin et al., 1971).

Accordingly, there is a great need for a method of reproducably and reliably regenerating Cucumis sp. plants by tissue or cell culturing techniques. This is the key to a successful adaptation of novel breeding methodologies and genetic engineering techniques to agriculture to more quickly and selectively produce improved plants having desirable traits.

The process of the present invention involves production of plantlets by embryogenesis from tissue explants in either "solid" media, such as agar-solidified media of various viscosities, or cell suspension culture where the cells are suspended in a gently agitated liquid medium. For general background see U.S. Patent 4,548,901 (which is directed to a suspension culture process for production of legume plantlets), and references cited therein, particularly G.G. Henshaw et al., (1982).

# 0 262 971

## THE INVENTION

### Objects

It is among the objects of this invention to provide processes for the in vitro generation (induction) and regeneration of embryoids and plantlets from explant tissue which are capable of developing into mature plants.

It is another object to provide novel media for the induction of embryogenesis and production of embryoids, which are are capable of development to fully mature plants.

It is another object of this invention to provide methods of continuously producing plant embryoids for encapsulation as artificial seeds.

It is another object of this invention to apply the novel methods and media of this invention to the production of improved plants in the genus Cucumis.

It is another object of this invention to shorten the time for producing genetically engineered and/or hybridized plant stock without having to go through full seed development.

It is another object of this invention to asexually propagate plants without hormonal or chemical induction of flowers.

It is another object of this invention to provide a tissue or cell culturing system which may be used in conjunction with various methods of introducing foreign genes into Cucumis sp.

It is another object of this invention to induce embryoids for frozen storage followed by thawing and continuation of maturation and development into plantlets and/or plants.

Still other objects of this invention will be evident from the balance of this specification and claims.

### Definitions

The terminology used herein is not intended to vary from the terminology used in the field. However, the meaning of some terms used in the field is not necessarily uniform, and the following definitions will be of help in this case:

"Plantlet" refers to a plant sufficiently developed to have a shoot and a root that is asexually reproduced by cell culture.

"Explant" refers to a section or a piece of tissue from any part of a plant or plantlet for culturing, whether wild or cultivated, hybrid (somatic or sexual), or genetically variant or transformed.

"Hormone" refers to a plant growth regulator that affects the growth or differentiation of plants, and is exogenous as used in reference to the various media herein.

"Callus", and its plural "calli" refer to an unorganized group of cells formed in response to a cut, severing or injury of a plant, and herein refers to the unorganized cell growth which may form on explant tissues during culturing.

"Embryoid" refers to a structure similar in appearance to plant zygotic embryo.

"Encapsulation" includes covering with one or more coatings, or placing bare or coated in empty or media-filled capsules.

Abbreviations:

NAA = alpha-naphthaleneacetic acid
BAP = 6-benzylaminopurine
2,4-D = 2,4-dichlorophenoxyacetic acid
MS Medium - Murashige and Skoog medium (Murashige and Skoog, 1962)
TM-1 - Tomato Media TM-1 (Shahin, 1985)
CTM = Cucumis Transformation Medium

### Summary:

We have discovered that a series of two or three media containing two or more hormones selected from the group comprising NAA, BAP, and 2,4-D are able to induce the development of somatic embryoids from explant tissue of the genus Cucumis in a variety of growth media ranging from tissue culture on solid (agar) substrates to cell suspension techniques.

All three hormones are contained in a modified MS medium which is the initial induction medium, medium CTM-2. Thereafter, the induced tissue is transferred to an embryoid expression (maturation) medium, medium CTM-3, containing NAA and BAP. The embryoids developed at this stage range in size from 2 mm to 1 cm in length. Finally the fully mature embryoids are transferred to a plantlet developmental medium, medium CTM-4, which is hormone free and promotes shoot elongation, rooting and normal plant development. The hormones are present in the media in the range of from about 0.5 to about 10 $\mu$M, preferably about 2 to about 5 $\mu$M.

The initial condition for the induction and expression phases include continuous light and temperature around 20-30°C, preferably 26-28°C. During the developmental phase, the photoperiod may be shortened to 10 - 20 hours of light, preferably 15 - 17 hours.

The methods of this invention permit regeneration of plants from any Cucumis sp. explant tissue whether wild or cultivated, hybrid (somatic or sexual), or genetically variant or transformed plants or plantlets. Thus, by the use of the methods and media of this invention it becomes unnecessary to treat gynoecious or androecious plants of the genus Cucumis with external hormones or chemical substances to induce male and/or female flowers. The current practice calls for such induction, followed by pollination and seed

3

production.

By the practice of this invention, field-ready plants can be produced in 70 - 100 days from explant selection as compared to six months for production of seed from seed-derived plants. The advantages are particularly significant in hybridization. For example, conventional known techniques involve the steps of development of inbred (parental) lines, of cross pollination, development of F-1 seed followed by the planting of F-1 seed. The maintenance of the parental lines and the making of the F-1 cross is very expensive and time consuming.

However, by the techniques of the present invention involving plant propagation in vitro, hybrid (F-1) plantlets can be reproduced within 10 weeks, with no need to propagate the parental lines and/or to continue making the crosses. The media of this invention permit a full range of expresion of somaclonal variations in wild or cultivated plant tissue, and may also be used following transformation.

By the method of this invention we have regenerated the representative Cucumis lines set forth in Table I below:

## TABLE I

| Cultivar or breeding line[1] | Type |
|---|---|
| 1. Marketmore 72F | cucumber, gynoecious, slicer |
| 2. W 1983 G | cucumber, gynoecious, pickle |
| 3. Sunblest Burpless | cucumber, monoecious, slicer |
| 4. Clinton | cucumber, monoecious, pickle |
| 5. W 2091 | cucumber, hermaphrodite |
| 6. Lemon Cucumber | cucumber, hermaphrodite |
| 7. W 744 GP | cucumber, parthenocarpic, pickle |
| 8. W 1082 HP | cucumber, parthenocarpic |
| 9. Bush Slicer | cucumber, determinate, slicer |
| 10. M 27 | cucumber, determinate, pickle |
| 11. Expo | cucumber, monoecious, slicer |
| 12. GY 14 | cucumber, gynoecious, slicer |
| 13. Wisconsin SMR 18 | cucumber, monoecious, slicer |
| 14. Straight Eight | cucumber, monoecious, pickle |
| 15. Hale's Best No. 36 | muskmelon, cantaloupe |
| 16. Rocky Ford, Green Flesh | muskmelon, cantaloupe |

1) Seeds obtained from ARCO Seeds Co., Brooks, Oregon, except for seeds of cucumber cultivar Expo, which was obtained from De Ruiter Zonen BV, Bleiswijk, the Netherlands. Explants from 12 seedlings in each lot were regenerated.

These Cucumis cultivars regenerated by the methods of this invention represent the principal sex types, growth habits, fruiting characteristics and processing qualities used in commercial breeding.

The regeneration methods and media of this invention were also tested on other members of the Cucurbitaceae as set forth in the Examples. Cucumbers and muskmelons, both members of the same genus Cucumis, responded almost identically, whereas the watermelon (Citrullus sp.) and squash (Cucurbita sp.) did not. The formation of the green domes in watermelon, however, suggests that modification of the existing protocol (media and methods) could lead to succesful regeneration in this genus.

The positive response of all tested genotypes of cucumbers and the high yields of regenerated plantlets render the regeneration methods and media of this invention particularly useful in breeding. One significant application is the production of gynoecious populations by the use of the methods of this invention to replace the current expensive treatment with silver nitrate. Another important use of the invention is the development of somaclonal variations in the regenerated plants as a valuable source of diversity in plant material used in breeding. The regeneration procedures of this invention may be used for selection via screening techniques of individuals resistant to pathogens, toxic metals, pesticides and herbicides. The latter is of particular importance because cucumbers are very sensitive to herbicides. Thus, standard screening techniques at the CTM-4 stage will rapidly identify somaclonal variants exhibiting such resistances.

The application of genetic engineering in cucumber and muskmelon breeding is of special value. These two species are sexually incompatible. Conventional crosses and transfer of many desirable traits such as disease resistance have not been possible (Deakin et al., 1971). As set forth herein, and in our copending applications, such incompatibility barriers are removed for cucumbers and muskmelons. Our case 92/12, filed of even date herewith, sets forth gene transfer and plant tranformation techniques, while our copending case 91/13, filed of even date herewith, sets forth protoplast regeneration and somatic hybridization techniques. Gene transfer can be accomplished by the leaf disk method of transformation with <u>Agrobacterium tumefaciens</u> (Horch et al., 1985), or by transforming roots using <u>A. rhizogenes</u>, as set forth in our copending case 92/12. In addition to the ability to transfer horticulturally desirable genes into cucumber or muskmelon, genetic engineering procedures are particularly valuable in introduction of marker genes such as resistance to kanamycin or chloramphenicol. These markers facilitate somatic hybridization via protoplast fusion, thus removing the serility barriers in the genus <u>Cucumis</u> as set forth in our copending case 92/13. Thus, both the transformation and protoplast techniques of those copending cases can be employed in combination with the explant regeneration of this case.

Detailed Description of the Best Mode of Carrying Out the Invention

The following detailed description is by way of example and not by way of limitation of the principles of this invention, and has reference to specific examples in which genus <u>Cucumis</u> embryo generation (induction) and plantlet regeneration within the scope of this invention is described for cucumber (<u>Cucumis sativus</u> L.) and muskmelon (<u>Cucumis melo</u> L.) by way of example.

1. Preparation of media

A. Medium CTM-1, Embryogenic Callus Inducing Medium

MINERAL SALTS:

MS major elements

MS minor elements

| ORGANIC CONSTITUENTS: | amount/1 |
|---|---|
| Casein hydrolysate | 1.0g |
| Glycine | 2.5mg |
| m - inositol | 100mg |
| Nicotinic acid | 5mg |
| Pyridoxin HCl | 0.5mg |
| Thiamine HCL | 0.5mg |
| Folic acid | 0.5mg |
| Biotin | 0.05mg |
| Sucrose | 30.0g |
| Agar purified (Difco) | 7.0g |

HORMONES:

2,4-D (5 μM)    1.1 mg/1

BAP (2 μM)    0.452 mg/1

B. Medium CTM-2. Direct Embryoid Induction Medium

As in 1.A, and also contains 5 M NAA (0.932 mg/1).

C. Medium CTM-3. Embryoid Expression (Maturation) Medium

As in 1.B, but free of 2,4-D; i.e., the medium contains only BAP (2 μM) and NAA (5 μM).

D. Medium CTM-4. Plantlet Development Medium.

As in 1.A, but is free of all three hormones (2,4-D, NAA and BAP), and has 10g of agar per liter of medium.

E. All Media.

In all media the pH was adjusted to 5.8 prior to autoclaving for 15 minutes at 121°C. To minimize bacterial contamination the media were supplemented with 100 mg/1 of Cefatoxime (Calbiochem), added aspectically to the medium after autoclaving and cooling to approximately 40°C. The CTM-2, CTM-3 and CTM-4 contained 0.7%, 0.7% and 1% of purified agar (Difco Laboratories), respectively.

2. Preparation of Explant

A. Cucumber (Cucumis sativus L.) - Cotyledonary Explants

Seeds of cucumber cv. Straight Eight (ARCO Seed, Brooks, Oregon) are sterilized in 10% (v/v) Clorox (commercial bleach containing 5.25% sodium hypochlorite) with a drop of Tween 80 for 10 minutes, then rinsed 3x in sterile, distilled water and placed in sterile petri plates (ca 30 per plate) lined with moist Whatman #3 filter paper. To assure uniform and rapid germination of the seeds, plates are kept for 24 - 30 hours at 27°C, in the dark. Germinated seeds (radicle length ca 5mm) are placed aseptically in Magenta boxes (six seeds per box) containing ca 40 ml of TM-1 medium (Shahin, 1985) supplemented with 150 mg/l of Carbenicillin (Sigma), and incubated for four days in a growth chamber, at 21°C night, 26°C day, 14 hour photoperiod (4500 lux). When the seedings are green, but the cotyledons only partially unfolded, the Magenta boxes are placed for 24 hours in the dark, at room temperature. The explant tissue is then prepared by aseptically removing the seedlings and cutting out disks (5 mm in diameter) from the cotyledonary tissue using a #2 corkborer.

B. Muskmelon (Cucumis melo L.) - Cotyledonary Explants

Seeds of muskmelon cv. Hale's Best (ARCO Seed, Brooks, Oregon) are germinated and the cotyledonary explants are prepared as in 2.A.

C. Cucumber (Cucumis sativus L.) - Root Explants

One centimeter long apical sections of the roots are taken from the seedlings described in 2.A.

3. Examples

Example A. Regeneration of cucumber plants from cotyledonary explants

1. Induction of competent embryoids

Explants prepared aseptically as in 2.A are cultured by placing them abaxial side down in petri plates containing medium CTM-2. The plates are sealed with parafilm and incubated in clear plastic boxes; in all examples, embryo induction, maturation and plantlet development were carried out at 27°C under continuous light (Sylvania, Cool White) at 3500 lux, unless otherwise noted. Small (1-2 mm in size), glossy embryoids form on the cut edges or on the surface of the cotyledonary explants after two to three weeks. They are yellowish-green in color, and are either separate, or form smooth-surfaced clusters. Some genotypes will first develop callus before forming embryoids.

If the explant tissue is left on this medium for a period of time longer than three weeks, callus will develop and the embryoids will die.

2. Promotion of the embryoid maturation (expression)

To promote further embryoid development and maturation the competent embryoids are separated with a minimum of surrounding tissue and transferred onto CTM-3 medium and cultured for additional five to 10 days under the same conditions (27°C, continuous light). The embryoids enlarge, turn green, and develop visible leaf structures. The root pole may be somewhat suppressed, but will develop (see below).

The embryoids should not be left on this medium past about 14 to 18 days, as callus will form and the embryoids will die.

3. Plantlet development

The mature embryoids are transferred onto CTM-4 medium where they are cultured at 27°C and photoperiod of 16 hours. Well developed cucumber plantlets are obtained in approximately two to six weeks, typically in about 30 days. The shoots elongate and normal plant development is observed. Callus may have to be excised from time to time on some of the embryoids.

Some secondary and tertiary embryoids are usually formed during this step of plantlet regeneration, and may be directly regenerated into plantlets on the CTM-4 Medium. With a large initial explant population, the process is substantially continuous and steady state, and needs no new starting material (explants).

4. Final development

The plantlets are placed on an acclimatizing solid agar medium, using medium CTM-4 diluted 50/50 with water, and grown to develop a good root system. They are then potted in soil. The regenerated plants proceed to final development being normal in physiology and morphology, and are fertile, produce fuit and germinable seed.

Example B. Regeneration of cucumber plants from cotyledonary callus

1. Induction of embryogenic callus

Explants prepared aseptically as in 2.A are cultured by placing them abaxial side down in petri plates containing medium CTM-1. The plates are sealed with parafilm and incubated in clear plastic boxes in a 27°C incubator under continuous light. After about 20 days brownish callus develops on the cut edges or in the vein areas of the explants.

2. Induction of competent embryoids

The callus is cut from the remaining necrotic tissue and transplanted onto fresh CTM-1 medium. After additional 15 to 20 days of culturing in CTM-1 medium under the same conditions as B-1 above, glossy, yellowish clusters of embryoids appear on the surface of the callus.

3. Promotion of the embryoid maturation

To promote further embryoid development and maturation the competent embryoids are separated with a minimum of surrounding callus and transferred onto CTM-3 medium and cultured for additional five to 10 days under the same conditions as in Example A. The embryoid maturation proceeds as in Example A.

4. Plantlet regeneration

Plantlet regeneration proceeds as in Example A.

5. Final development

Final development proceeds as in Example A.

The induced embryogenic callus on medium CTM-1 (step 2) will continue to produce competent embryoids when subcultured periodically on the same medium. Thus a continuous steady state production of competent embryoids can be obtained without introduction of additional explants.

The results of example A using medium CTM-2 to generate competent embryoids are better than Example B. In Example A up to 100% of the explants produces competent embryoids which develop into normal plants. Approximately three to 20 competent embryoids or embryoid clusters are produced per explant. In Example B the regeneration process is slower and more abnormalities develop.

Example C. Regeneration of cucumber plants from root explants

1. Induction of competent embryoids

Root explants are prepared aseptically as in 2.C and placed in petri plates containing medium CTM-2. The plates are sealed with parafilm and incubated in clear plastic boxes in a 27°C incubator under continuous light. Small, glossy embryoids form on the surface of the cultured root tissue after three to four weeks. They are yellowish-white in color, and are either separate, or form smooth-surfaced clusters.

2. Promotion of the embryoid maturation

To promote further embryoid development and maturation the competent embryoids are separated from the root tissue and transferred onto CTM-3 medium and cultured for additional five to 10 days under the same conditions as in Example A. The embryoid maturation proceeds as in Example A.

3. Plantlet regeneration

Plantlet regeneration proceeds as in Example A.

4. Final development

Final development proceeds as in Example A.

Example D. Regeneration of muskmelon plants from cotyledonary explants.

1. Induction of competent embryoids

Explants prepared aseptically as in 2.B are cultured by placing them abaxial side down in petri plates containing medium CTM-2. The induction of competent embryoids proceeds under the conditions of and as in Example A.

2. Promotion of the embryoid maturation

The promotion of embryoid maturation proceeds as in Example A.

3. Plantlet regeneration

The mature embryoids are transferred onto CTM-4 medium where they are cultured two to six weeks at the same conditions as in A.1. Well developed Cucumis melo plantlets may be obtained approximately in about 30 days, however, frequently two or three transfers every three to six weeks on medium CTM-4 and removal of surrounding callus is necessary to obtain normal plants.

4. Final development

The final development proceeds as in Example A.

Example E. Cell suspension process

Viable embryoids may be produced by cell suspension techniques employing the media of this invention. Explants from single genotypes or from somatic hybrids should be placed on agar-solidified CTM-1 Medium to promote callus formation as in Example B-1. The resulting embryogenic callus should then be transferred to a liquid CTM-1 Medium in a flask to promote competent embryoid formation. The flask is gently shaken between 50 - 150 cycles per minute under culturing conditions set forth above in Example B-1. Alternatively the culturing may be carried out in the dark at 27°C until the competent embryoids are formed (up to about 20 days). The CTM-1 Medium used here is the same as used in Example B, but no agar is used to solidifying the medium.

The calli should break into small aggregates which then should form competent embryoids. The resulting embryoids should then be successively transferred to flasks containing Medium CTM-3 (with agar for solid medium propogation, or without agar as a continuation of cell suspension culturing), and thence to Medium CTM-4 solidifed with agar for the periods called for above in Example A. The remaining undeveloped calli may be maintained by serial subculture in continuously refreshed Medium CTM 1 (without agar) to generate a substantially continuous flow of embryoids to development and expression in Media CTM-3 and CTM-4.

In the alternative, the liquid CTM-1 Medium may be flushed from the flask leaving freshly generated competent embryoids. The CTM-1 Medium would be replaced by fresh CTM-3 Medium for the requisite development step of the retained embryoids. At the end of the required period described above, fully developed embryoids may then be transferred to encapsulation, or to solid CTM-4 media for plantlet development, thence to rooting or hardening medium, and finally to soil.

Example F. Embryoid encapsulation -Production of artificial seeds.

Since in vitro production of somatic embryoids results in embryoids without protective seed coats, the embryoids may be encapsulated, individually or in groups, in capsules or coatings to retard dehydration and preserve them for future "planting". The capsules or coating may be any suitable composition of insoluble material or a slowly soluble gelatine or polymer composition, preferably opaque or semi-opaque, and preferably containing development retardant(s) to block precocious germination (further development). The capsules may be empty or contain a medium-hard agar with the appropriate medium therein.

For the capsule embodiment, we prefer to coat or embed mature embryoids in a CTM-4 Medium containing from $10^{-5}$ to $10^{-7}$M ABA (abscisic acid) hardened with agar. Gelatine capsules can then be filled with a mix of the embryoids and agar/CTM-4/ABA. In the alternative, the agar/CTM-4/ABA-coated embryoids may be over-coated with 2 - 5% polyethylene oxide solution, and dried on teflon or paper sheets or in suitably-sized screen interstices.

In the alternative, the bare embryoids can be directly drop-coated with polyethylene oxide (.5 - 10% solutions) to form a continuous coating thereon when dry. The ABA may be added to the polyethylene oxide solution.

In addition, the mature embryoids developed in the CTM-3 solution may be subjected to a retardation pre-treatment in an ABA solution for from 3 - 7 days before the encapsulation.

The thus-encapsulated embryoids may be "grown" as follows. In the case of the insoluble capsules, the capsules may be shipped and opened on site with the embryoids (coated or bare) implanted on plates containing either Medium CTM-3 or CTM-4 from which the development of rooted plantlets/plants should occur as above-described. The plantlets/plants are then transplanted to fields. In the case of the soluble capsules and polyethylene oxide-coated embryoids, they may be sown in agar containing Medium CTM-3 or CTM-4 or sown directly into rooting medium such as "Plant-Lite" with appropriate nutrients. Upon contacting the moistened agar or the wet rooting medium, the capsules and/or coating(s) will dissolve, the ABA will be diluted to ineffectiveness and the embryoids will be released to continue development into plantlets/plants as above-described.

Example G. Regeneration after Freeze Storage.

One or more of the explant sources, explants therefrom, or embryoids (at various stages, including in encapsulated form) may be stored frozen (dry ice, or preferably liquid nitrogen) for extended periods, then thawed and regenerated following the steps and media set forth above. The embryoids may be coated with agar-hardened CTM-4 Medium prior to freezing.

This technique of freezing competent embryoids provides a convenient genetic information storage method. Any of the well-known, conventional freezing, storage and thawing techniques for tissue, cells (e.g. sperm banks), and cell cultures (e.g. ATCC type cultures) may be employed in combination with the novel media and methods herein.

It should be understood that various modifications within the scope of this invention can be made by one of ordinary skill in the art without departing from the spirit thereof. We therefore wish our invention to be defined by the scope of the appended claims as broadly as the prior art will permit, and in view of this specification if need be.

References:

1. Carlson, P. (1973): Methione sulfoximine - resistant mutants of tobacco. Science 180, 1366-1368.

2. Chaleff, R. S., and Parsons, M. F. (1978): Direct selection in vitro for herbicide-resistant mutants of Nicotiana tabacum. Proc. Nat. Acad. Sci (USA) 75, 5104-5107.

3. Deakin, J.R., Bohn, G.W., and Whitaker, T.W. (1971): Interspecific Hybridization in Cucumis. Econ. Bot. 25, 195-211.

4. Henshaw, G.G., et al (1982): Morphogenic Studies in Plant Tissue Cultures. In: Differentiation in Vitro. M.M. Yeoman and D.E.S. Truman, eds., Cambridge Press.

5. Jelaska, S. (1972): Embroyd formation by fragments of cotyledons and hypocotyls in Cucurbita pepo. Planta 103, 278-280.

6. Jelaska, S. (1974): Embryogenesis and organogenesis in pumpkin explants. Phsiol. Plant. 31, 257-261.

7. Larkin, P.J., and Scowcroft, W.R. (1981): Somaclonal Variation - a Novel Source of Variability from Cell Cultures for Plant Improvement. Theor. Appl. Genet. 60, 197-214.

8. Malepszy, S., and Nadolska-Orczyk, A. (1983): In vitro culture of Cucumis sativus I. Regeneration of Plantlet from Callus Formed by Leaf Explants. Z. Pflanzenphsiol Bd. 111, 273-276.

9. Moreno, V., Garcia-sogo, M., Granell, I., Garcia-Sogo, B., and Roig, L.A. (1985): Plant Regeneration from Calli of Melon (Cucumis melo, L., cv. "Amarillo Oro"). Plant Cell Tissue Organ Culutre 5, 139-146.

10. Murashige, T., and Skoog, F. (1962): A revised medium for rapid growth and bioassays with tobacco tissue cultures. Physiol. Plant 15, 473-479.

11. Novak, J., and Dolezalova, M. (1982): Hormone control of growth and differentiation in the in vitro cultured tissue of cucumber (Cucumis sativus, L.). Biologia (Bratislava) 37, 283-289.

12. Shahin, E. A. (1985) Totipotency of tomato protoplasts. Theor. Appl. Genet. 69, 235-240.

13. Wehner, T., and Locy, R.D. (1981): In Vitro Adventitious Shoot and Root Formation of Cutivars and Lines of Cucumis sativus L. HortScience 16, 759-760.

14. Wenzel, G. (1985). Stategies in unconventional breeding for disease resistance. Ann. Rev. Phytopathol. 23, 149-172.

15. Lazarte, J. E. and Sasser, C. C. 1982. Asexual embryogenesis and Plantlet Development in Anther Culture of Cucumis sativus L. HortScience 17:88.

**0 262 971**

16. Sekioka, T. T. and Tanaka, J. S. 1981. Differentiation in callus cultures of cucumber (Cucumis sativus L.) Abstract.

17. Kim, Sang-Gu and Jang, Joung-Ran. 1984. Regeneration of plants from callus tissue of cucumber (Cucumis sativus L.) seeding cotyledons. Abstract, Plant Physiol. 75:15.

18. Kitto, S. L. and Janick, J. (1985). Production of Synthetic Seeds by Encapsulating Asexual Embryos of Carrot. J. Am. Socy. Hort. Sci. 110(2):227-282.

## Claims

1. Method of generation of competent Cucumis sp. embryoids, mature embryoids, or plantlets from Cucumis sp. plant tissue comprising the steps of:
    a) culturing explants of Cucumis sp. tissue in an induction medium containing exogenous hormones selected from the group consisting of:
    i) BAP and 2,4-D, and
    ii) NAA, BAP and 2,4-D,
    for a time sufficient to induce competent embryoids and
    b) optionally culturing said competent embryoids in an expression medium containing exogenous hormones consisting essentially of NAA and BAP for a time sufficient to promote further development and maturation to produce mature embryoids and further
    c) optionally culturing the mature embryoids in a plantlet regeneration medium essentially free of said hormones NAA, BAP, and 2,4-D for a time period sufficient to develop a plantlet.

2. The method of claim wherein:
said hormones are present in said induction medium in a concentration in the range of from about 0.5 to about 10 μM;
said induction culturing is maintained in a temperature range of from about 20-30° C, and
said induction culture is maintained under a photoperiod of continuous light.

3. The method as in claim 1 wherein:
    a) said induction medium contains BAP and 2,4-D, and is substantially free of NAA;
    b) said induction culturing is continued for a time sufficient to develop embryogenic callus; and
    c) said embryogenic callus is removed and transferred to fresh induction medium for an additional induction period to produce competent embryoids on embryogenic callus fully induced during an additional induction period; and
    d) optionally periodically subculturing said fully induced embryogenic callus in said BAP and 2,4-D induction medium to substantially continuously produce competent embryoids.

4. The method as in claim 3 wherein:
said hormones are present in said induction medium in a concentration in the range of from about 0.5 to about 10 μM;
said induction culturing is maintained in a temperature range of from about 20-30° C; and
said induction culture is maintained under a photoperiod of continuous light.

5. The method of claim 1 wherein:
during said step of plantlet regeneration, secondary or tertiary embryoids are formed; and wherein
said secondary or tertiary embryoids are transferred into and cultured in said plantlet regeneration medium to substantially continuously produce competent embryoids.

6. The method as in claim 5 wherein:
said plantlet regeneration culturing is
maintained in a temperature range of from about 20-30° C;
maintained under a photperiod of continuous light or maintained under a photoperiod in the range of from about 10 to about 20 hours of light; and
maintained for a period of from about two to six weeks.

7. The method as in claim 1 wherein said plant tissue is from the species Cucumis sativus L. or Cucumis melo L.

8. Competent Cucumis sp. embryoids produced by the process of claim 1.

9. Competent Cucumis sp. embryoids of claim 8 encapsulated as artificial seeds.

10. A method to culture Cucumis sp. embryoids which comprises
thawing frozen competent embryoids prepared by the process of claim 1 and frozen; and
culturing said thawed embryoid to promote further development and maturation.

11. The method of claim 10 wherein said culturing step comprises:
culturing said embryoids in an expression medium containing exogenous hormones consisting essentially of NAA and BAP for a time sufficient to promote further development and maturation, optionally with the added step of:
culturing the mature embryoids in a plantlet regeneration medium essentially free of said hormones NAA, BAP, and 2,4-D for a time period sufficient to develop a plantlet.

12. A culture medium selected from the group consisting of CTM-1, CTM-2, CTM-3 and CTM-4.